# EUROPEAN PATENT APPLICATION

(11) **EP 3 721 723 A1**
(43) Date of publication of application: **14.10.2020**
(21) Application number: 19169007.2
(22) Date of filing: 12.04.2019
(51) Int. Cl.: A24B 15/16, A24F 47/00, A61K 31/465

(54) **A CARRIER AND A METHOD OF USE THEREOF**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A carrier, and method of using the carrier, for use in a vaping smoking substitute device. The carrier including a substrate enclosing a plant material, wherein the substrate comprises a nicotinic compound, and wherein the substrate is configured to release the nicotinic compound into an aerosol-former liquid upon immersion in the aerosol-former liquid. The method of using the carrier including combining the carrier with an aerosol-former liquid; and allowing the nicotinic compound to disperse in the aerosol-former liquid.

## Description

### Field of the Invention

The present invention relates to a carrier for use in a vaping smoking substitute device, and method of use thereof.

### Background

Smoking substitute devices, which may also be known as electronic nicotine delivery systems, may comprise electronic systems that permit a user to simulate the act of smoking by producing an aerosol, also referred to as a "vapour", which is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and tobacco products.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach.

One approach for a smoking substitute device is the so-called "vaping" approach, in which a vaporisable liquid, typically referred to as "e-liquid", is heated by a heating device to produce an aerosol vapour which is inhaled by a user. An e-liquid typically includes a base liquid as well as nicotine and/or flavourings. The resulting vapour therefore typically contains nicotine and/or flavourings.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices which typically have a sealed tank and heating element which is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, the main body can be reused by connecting it to a new consumable. Another subset of closed system vaping smoking substitute devices are completely disposable, and intended for one-use only.

There are also "open system" vaping smoking substitute devices which typically have a tank that is configured to be refilled by a user, so the device can be used multiple times.

An example vaping smoking substitute device is the myblu™ e-cigarette. The myblu™ e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick which is partially immersed in the e-liquid. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute device is the blu PRO™ e-cigarette. The blu PRO™ e cigarette is an open system device which includes a main body, a (refillable) tank, and a mouthpiece. The main body and tank are physically and electrically coupled together by screwing one to the other. The mouthpiece and refillable tank are physically coupled together by screwing one into the other, and detaching the mouthpiece from the refillable tank allows the tank to be refilled with e-liquid. The device is activated by a button on the main body. When the device is activated, electrical energy is supplied from the power source to a heating device, which heats e-liquid from the tank to produce a vapour which is inhaled by a user through the mouthpiece.

Another approach for a smoking substitute device is the so-called "heat not burn" ("HNB") approach in which tobacco (rather than e-liquid) is heated or warmed to release vapour. The tobacco may be leaf tobacco or reconstituted tobacco. The vapour may contain nicotine and/or flavourings. In the HNB approach the intention is that the tobacco is heated but not burned, i.e. does not undergo combustion.

A typical HNB smoking substitute device may include a main body and a consumable. The consumable may include the tobacco material. The main body and consumable may be configured to be physically coupled together. In use, heat may be imparted to the tobacco material by a heating device that is typically located in the main body, wherein airflow through the tobacco material causes moisture in the tobacco material to be released as vapour. A vapour may be formed from a carrier in the tobacco material and additionally volatile compounds released from the tobacco. The released vapour may be entrained in the airflow drawn through the tobacco.

As the vapour passes through the smoking substitute device (entrained in the airflow) from an inlet to a mouthpiece (outlet), the vapour cools and condenses to form an aerosol (also referred to as a vapour) for inhalation by the user. The aerosol will normally contain the volatile compounds.

An example of the HNB approach is the IQOS® smoking substitute device from Philip Morris Ltd. The IQOS® smoking substitute device uses a consumable, including reconstituted tobacco located in a wrapper. The consumable includes a holder incorporating a mouthpiece. The consumable may be inserted into a main body that includes a heating device. The heating device has a thermally conductive heating knife which penetrates the reconstituted tobacco of the consumable, when the consumable is inserted into the heating device. Activation of the heating device heats the heating element (in this case a heating knife), which, in turn, heats the tobacco in the consumable. The heating of the tobacco causes it to release nicotine vapour and flavourings which may be drawn through the mouthpiece by the user through inhalation.

A second example of the HNB approach is the device known as "Glo"® from British American Tobacco p.l.c. Glo® comprises a relatively thin consumable. The consumable includes leaf tobacco which is heated by a heating device located in a main body. When the consumable is placed in the main body, the tobacco is surrounded by a heating element of the heating device. Activation of the heating device heats the heating element, which, in turn, heats the tobacco in the consumable. The heating of the tobacco causes it to release nicotine vapour and flavourings which may be drawn through the consumable by the user through inhalation. The tobacco, when heated by the heating device, is configured to produce vapour when heated rather than when burned (as in a smoking apparatus, e.g. a cigarette). The tobacco may contain high levels of aerosol formers (carrier).

A host of e-liquid formulations are commercially available for use in vaping smoking substitute devices.

Generally e-liquids do not contain solid material as this can interfere with the capillary action of the wick and cause problems with clogging. There have been prior proposals for enabling a consumer to infuse base liquid with solid material in order to customise the flavour of the e-liquid. However, the intention is that solid material is removed prior to supplying the e-liquid to a device for vaporization.

Although vaping smoking substitute devices are gaining popularity in many parts of the world, they are not universally acceptable for use and in a significant number of countries the sale or supply of nicotine-containing e-liquids is restricted. This may be due to the fact that in many countries purified nicotine is classified as a pharmaceutical product and for this reason the sale and use of nicotine-containing liquids is highly regulated. Such countries may in fact prohibit the sale of nicotine-containing e-liquids.

In some countries vaping smoking devices are considered to be drug delivery devices and consequently their use and / or sale may also be prohibited.

It would be desirable to provide a vaping smoking substitute device and system which can be used to deliver an aerosol containing a nicotine component and which would be acceptable for sale and use in countries which currently do not permit the sale and use of purified nicotine-containing e-liquids due to their classification as drugs or pharmaceutical products.

In particular, it would also be highly desirable to provide a vaping smoking substitute system which does not require the consumer to purchase a purified nicotine-containing liquid. It would also be desirable to provide a vaping smoking substitute system which does not require the consumer to handle a purified nicotine-containing liquid.

### Summary of the Invention

The present invention relates a carrier and method of use thereof. In particular, the carrier is of use in a vaping smoking substitute device.

According to a first aspect of the present invention there is provided a carrier for use in a vaping smoking substitute device, the carrier comprising a substrate enclosing a plant material,
wherein the substrate comprises a nicotinic compound, and wherein the substrate is configured to release the nicotinic compound into an aerosol-former liquid upon immersion in the aerosol-former liquid.

As used herein, the term "nicotinic compound" or "nicotinic" is intended to refer to nicotine, nicotine salt(s), nicotine complex(es) and/or nicotine solvate(s).

As used herein, the term "plant material" is intended to refer to a portion and/or part(s) of a plant (e.g. leaf, stem, flower or bud). The plant material may be processed (for example, by shredding, grinding or drying) or it may be non-processed (that is, used whole). The plant material is typically fibrous (comprising or characterised by fibres). For the avoidance of doubt the term "plant material" is not intended to include pulp and/or paper which is derived from a plant material and chemically and/or mechanically processed to extract fibres before use.

In this way, a controlled dose of nicotinic compound can be delivered to the aerosol-former liquid. The aerosol-former liquid may be referred to as an e-liquid. In the context of vaping this provides the user with an improved sensory experience. The dose of the nicotinic compound may also be tailored to suit the user's requirements. Additionally, it allows the nicotinic compound to be efficiently and rapidly delivered to the aerosol-former liquid. Also, the plant material, in itself, may naturally contain an active compound. Consequently, as the carrier comprises a nicotinic compound the number/concentration of active compounds in the vapour produced by the aerosol-former liquid is increased. This provides the user with an enhanced recreational and/or pharmaceutical effect.

Examples of active compounds include, but it not limited to, nicotinic compounds (e.g. nicotine), cocaine, caffeine, opiates and opioids, cathine and cathinone, kavalactones, mysticin, beta-carboline alkaloids, salvinorin A together with any combinations, functional equivalents to, and/or synthetic alternatives of the foregoing.

Preferably, the substrate is fluid-permeable. Alternatively, the substrate may become fluid-permeable on immersion in the aerosol-former liquid.

Preferably, the nicotinic compound comprises nicotine.

Advantageously, this provides the user with an enhanced nicotine "hit". Also, nicotine provides the user with an enhanced "throat hit" (i.e. the feeling the user gets on the back of their throat when inhaling the aerosol produced by an aerosol-former liquid).

Preferably, the nicotinic compound comprises a nicotine salt.

Advantageously, nicotine salts exhibit higher stability and lower volatility than nicotine. This is advantageous for storage and processing of the carrier. For example, the carrier can more safely be handled. Also, the carrier will have an improved shelf life. The use of nicotine salts also provides a product with smoother "throat hit" when used in a vaping smoking substitute device.

Preferably, the nicotine salt is selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

Advantageously, these salts provide the user with a satisfactory nicotine "hit" and additionally provide an improved sensory experience. In addition, these salts have good solubility in many aerosol-former liquids and therefore allow rapid release of the nicotine salt in the aerosol-former liquid.

Any suitable plant material that may be used within a vaping smoking-substitute device may be used in the present invention.

The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

Preferably, the plant material comprises tobacco. In one embodiment, the plant material is tobacco.

Advantageously, as the invention is primarily intended for use in a vaping smoking substitute device it is desirable to replicate the qualities of conventional smoking devices, this is achieved by using tobacco. This allows the user to closely replicate the smoking experience as the aroma, flavour and sensation inherent to tobacco is provided.

Preferably, the plant material is a freeze-dried plant material.

Advantageously, this provides a product with a longer shelf-life.

Preferably, the nicotinic compound is coated onto the substrate. The coating may be a composition comprising the nicotinic compound.

Advantageously, a coating ensures a more consistent and homogenous distribution of the nicotinic compound on the carrier. This provides a more consistent and improved sensory experience to the user. Suitable methods of coating include spray-coating, brush coating, and dip coating.

Preferably, the substrate comprises a water-insoluble film containing the nicotinic compound. In particular, the substrate comprises a water-insoluble film containing the nicotinic compound, wherein the water-insoluble film does not dissolve in the aerosol-former liquid.

Advantageously, this overcomes the problems associated with water-soluble films. In some instances aerosol-former liquids for vaping smoking substitute devices contain water. Consequently, a water soluble film would be disadvantageous as the solubilised film components may end up in the vapour, and subsequently inhaled by the user, or they may interfere with the device and/or vaping process. For the same reasons, the water-insoluble film is not soluble in the aerosol-former liquid. The nicotinic compound contained on/within the water-insoluble film may diffuse from the water-insoluble film into the aerosol-former liquid, such that the water-insoluble film does not itself substantially dissolve into the aerosol-former liquid and/or water.

Preferably, the carrier is a pouch.

The pouch may be formed from fiber, paper, cloth, and fabric. The pouch may be formed from one or more polymeric materials. The polymeric material may comprise one or more of hydroxypropyl cellulose (HPC), hydroxypropyl methylcellulose (HPMC), polyvinyl alcohol (PVOH), polyvinylpyrrolidone (PVP), polyethylene oxide (PEO) hydroxyethyl cellulose (HEC), polyethylene glycol (PEG), pullulan, sodium alginate, xanthan gum, tragancanth gum, guar gum, acacia gum, arabic gum, polyacrylic acid, maltodextrin, methylmethacrylate copolymer, carboxyvinyl copolymers, starch and gelatin. Advantageously, a pouch has suitable qualities for storage and use. In particular, a pouch provides an aesthetic quality that is satisfactory to the user. The pouch provides an arrangement similar to that used for teabags.

Preferably, the aerosol-former liquid comprises a polyhydric alcohol.

Advantageously, good dispersion of the nicotinic compound is achieved in polyhydric alcohols.

Suitable polyhydric alcohols include propylene glycol (PG), triethylene glycol, 1,2-butane diol and vegetable glycerine (VG)) and their esters (e.g. glycerol mono-, di- or tri-acetate).

It is most preferred that the polyhydric alcohol is propylene glycol or vegetable glycerine or combinations thereof. Advantageously, propylene glycol or vegetable glycerine exhibit minimal toxicity. Furthermore, they impart a sweet taste that is satisfactory to the user.

According to a second aspect of the invention there is provided a method of using of the carrier according to the first aspect comprising:
combining the carrier with an aerosol-former liquid; and
allowing the nicotinic compound to disperse in the aerosol-former liquid.

Advantageously, this facilitates rapid and efficient infusion of the aerosol-former liquid with the nicotinic compound.

Preferably, the method includes a step of agitating the carrier within the aerosol-former liquid.

Advantageously, this provides improved infusion and good dispersion of the nicotinic compound into the aerosol-former liquid.

Preferably, the method includes a step of heating the aerosol-former liquid.

Advantageously, this allows improved infusion of the nicotinic compound into the aerosol-former liquid.

According to a third aspect of the invention there is provided use of the carrier according to the first aspect of the invention in a vaping smoking substitute device.

According to the fourth aspect of the invention there is provided a kit comprising the carrier according to the first aspect, and an aerosol-former liquid.

According to a fifth aspect of the invention there is provided a vaping smoking substitute consumable comprising the carrier according to the first aspect of the invention.

According to a sixth aspect of the invention there is provided a vaping smoking substitute system comprising a vaping smoking substitute consumable according to the fifth aspect of the invention, and a vaping smoking substitute device.

### Summary of the Figures

So that the invention may be understood, and so that further aspects and features thereof may be appreciated, embodiments illustrating the principles of the invention will now be discussed in further detail with reference to the accompanying figures, in which:
**Figure 1****.** is a perspective view of a carrier in accordance with the teachings of the present invention.
**Figure 2****.** is an illustrative view of a manufacturing process for producing carriers for use in accordance with the present invention.
**Figure 3****.** is an illustrative view of the method of using the carrier according to the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

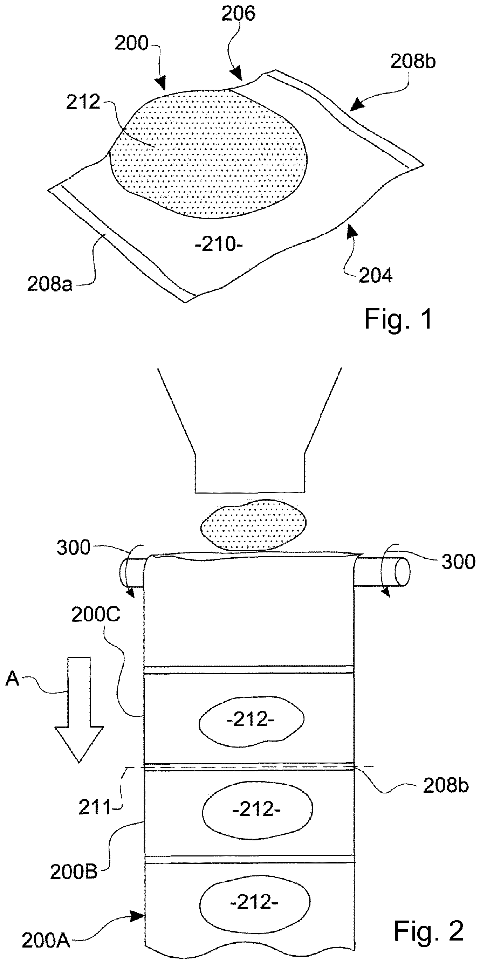

Figure 1 illustrates the preferred embodiment of the invention. Figure 1 shows a perspective view of an extraction bag or pouch 200. A main body of the pouch 200 has a generally polygonal shape. As shown in Figure 1 the main body is generally rectangular in shape. The main body has a first surface 204 and a second surface 206 and seams 208 formed around the external edges to define a pocket 210 therebetween. A quantity of a plant material is located in the pocket 210.

In the preferred embodiments of the invention that is illustrated in Figure 1, the main body is formed from a substrate having a pore size suitable for constraining the bulk material of the plant material to be constrained within the pocket 210. The substrate has a coating of a nicotinic compound. The coating may fully coat the substrate. Alternatively, the coating may only partially coat the substrate (e.g. it may only cover just one major surface of the substrate; a selected portion of the substrate; or it may be patterned on the substrate). In use, the pouch 200 is combined with an aerosol-former liquid. The aerosol-former liquid permeates the substrate of the pouch 200 to allow the active compound(s) present in the plant material to infuse throughout the aerosol-former liquid. At the same time the coating on the substrate infuses the nicotinic compound throughout the aerosol-former liquid.

When manufacturing the pouch 200 a sheet of continuous cylindrical material or sleeve coated with one or more nicotinic compounds is fed in the direction indicated by arrow A in Figure 2. The sleeve is held vertically and configured so that as the sleeve is fed over rollers 300. The surfaces of the sleeve separate to form the pocket 210 and is sealed along edge 208a. The sealing may preferably be achieved by the application of pressure and/or heat.

A measured quantity of the plant material 212 is then deposited through the open end 208b into the pocket 210 formed between the sealed opposing surfaces 204 and 206. The ratio of the quantity of plant material 212 and the dimensions of the pouch 200 are critical as medium that is too tightly packed within the pouch 200 may compromise the subsequent recovery of the active compounds contained in the plant material. The surfaces 204 and 206 are sealed along edge 208b to constrain the medium 212 within the pocket 210. Finally, once the surfaces are sealed along edge 208b the main body is cut away from the sheet or sleeve along edge 208b and just above the seal. As can be seen from Figure 2, the seal formed along the upper edge 208b of pouch 200a forms the lower seal 208a of the subsequent pouch 200b above. Consequently, the seal 208 between each pouch should be of sufficient thickness to permit the pouches 200a and 200b to be separated by cutting along the centre of the seal, along the line indicated by dotted line 211 whilst leaving the upper edge 208b of pouch 200a and lower edge 208a of pouch 200b sealed. Thus, horizontal sealing and cutting may be simultaneously performed.

Turning now to the main body 202 that is coated with one or more nicotinic compounds. In the embodiments of the invention illustrated in Figures 1-2 the nicotinic compound is nicotine. The coating can be applied to the main body by dip coating, spray coating, or vapour deposition methods. Such that when pouch 200 is combined with an aerosol-former liquid the coating will release the nicotine into the aerosol-former liquid. The coating can be in the form of a water-insoluble film containing nicotine. Such water-insoluble films may be polymeric in nature.

Turning now to the plant material 212 that is constrained within the pouch 200, in the embodiments of the invention illustrated in Figures 1-2, the bulk of the plant material 212 therein comprises tobacco. For the purposes of the present invention it is immaterial whether the tobacco is leaf or stem, or whether it is in shredded, ground, or powdered form provided that the pores of the substrate from which the surfaces 204 and 206 of the pouch 200 are formed are dimensioned to constrain the tobacco within the pouch 200.

The form of the tobacco is immaterial to the teachings of the present invention other than it should be appropriately dimensioned to fit within the pocket of the pouch 200. In a particularly preferred embodiments of the present invention the tobacco is in the form of dust or powder to provide the optimal surface area. The tobacco is typically pre-treated by grinding before being dried.

According to the teachings of the present invention the tobacco is first washed in purified water to remove contaminants. The process of washing the tobacco may cause the material to swell.

A composition of vegetable glycol and nicotine is then added to the tobacco. The vegetable glycol causes the nicotine to be more evenly distributed across the organic tobacco material. The intention is to saturate the tobacco with vegetable glycol rather than nicotine but it also enhances the saturation of the nicotine into the material. The composition is preferably in the ratio 1:3 nicotine to vegetable glycol and is added to 3 parts tobacco material.

The damp organic tobacco material may be freeze-dried before being deposited into the pouch 200. Each pouch will typically comprise 0.7 g tobacco material, 0.7 g of vegetable glycol, and greater or equal to 0.24 g of nicotine. However, it will be apparent to one of ordinary skill in the art that alternative compositions will be appropriate for different types and forms of organic tobacco material and fillers.

Once the pouch 200 has been sealed as described in the foregoing description, it is placed within a container 300 which is purged of air with a gas such as argon and sealed to prevent evaporation and degradation.

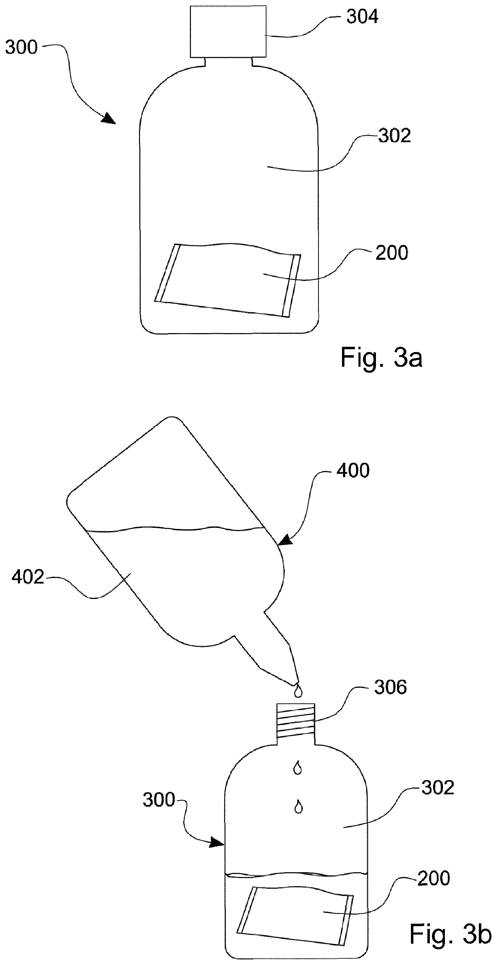

Referring to Figures 3a, 3b, immediately prior to requiring the aerosol-former liquid, the user removes the cap 304 from the neck of the container 300 to expose the aperture 306 and to provide access to the chamber 302 through the aperture 306 to the pouch 200 contained therein. A measured volume of fluid 402 is then dispensed from a dispenser 400 through the aperture 306 of container 300 and into the chamber 302. The measured volume of fluid is such that it does not completely fill chamber 302 but leaves a headspace of air above. The cap 304 is then replaced to seal the chamber with both the measured volume of fluid 402 and the pouch 200.

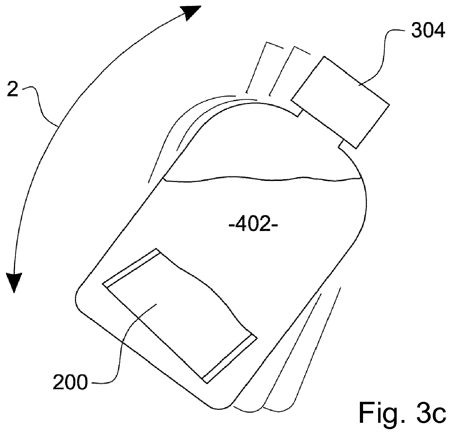

As shown in Figure 3c, next the user shakes the container 300 vigorously for period of 10 seconds, before allowing the container 300 to stand for a period of 10 minutes. Upon expiry of the period of 10 minutes the user then shakes the container for a further period of 10 seconds. Heat may also be applied to aid dispersion of nicotine into the aerosol-former liquid. The resultant formulation can then be used as whole or part of a consumable for a vaping smoking substitute device.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A carrier for use in a vaping smoking substitute device, the carrier comprising a substrate enclosing a plant material, wherein the substrate comprises a nicotinic compound, and wherein the substrate is configured to release the nicotinic compound into an aerosol-former liquid upon immersion in the aerosol-former liquid.

2. The carrier of claim 1, wherein the nicotinic compound comprises nicotine.

3. The carrier of any preceding claim, wherein the nicotinic compound comprises nicotine salt.

4. The carrier of claim 3, wherein the nicotine salt is selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

5. The carrier of any preceding claim, wherein the plant material comprises tobacco.

6. The carrier of any preceding claim, wherein the plant material is a freeze-dried plant material.

7. The carrier of any preceding claim, wherein the nicotinic compound is coated onto the substrate

8. The carrier of any preceding claim, wherein the substrate comprises a water-insoluble film containing the nicotinic compound.

9. The carrier of any preceding claim, wherein the carrier is a pouch.

10. The carrier of any preceding claim, wherein the aerosol-former liquid comprises a polyhydric alcohol.

11. A method of using the carrier according to any preceding claim comprising:
combining the carrier with an aerosol-former liquid; and
allowing the nicotinic compound to disperse in the aerosol-former liquid.

12. The method of claim 11 comprising:
a step of agitating the carrier within the aerosol-former liquid

13. The method of claim 11 or claim 12 comprising:
a step of heating the aerosol-former liquid.

14. Use of the carrier according to any of claims 1 to 10 in a vaping smoking substitute device.

15. A kit comprising the carrier according to any of claims 1 to 10 and an aerosol-former liquid.
